(19) Europäisches Patentamt
European Patent Office
Office européen des brevets

(11) Publication number: 0 271 679 A2

(12) **EUROPEAN PATENT APPLICATION**

(21) Application number: 87115748.3

(22) Date of filing: 27.10.87

(51) Int. Cl.4: C07C 118/00, C07C 119/042, C07C 125/065, C07C 125/073, C07D 239/10, C08L 33/14, C08L 67/00

(30) Priority: 03.11.86 US 926067

(43) Date of publication of application: 22.06.88 Bulletin 88/25

(84) Designated Contracting States: AT BE CH DE FR GB IT LI NL SE

(71) Applicant: AMERICAN CYANAMID COMPANY
1937 West Main Street P.O. Box 60
Stamford Connecticut 06904-0060(US)

(72) Inventor: Waterman, Paul Sheldon
15 Joseph Court
Shelton Connecticut 06484(US)
Inventor: Feldman, Allan M.
4 Hunters Lane
Norwalk Connecticut(US)

(74) Representative: Wächtershäuser, Günter, Dr.
Tal 29
D-8000 München 2(DE)

(54) **Isocyanates derived from dienes and process and their preparation.**

(57) A process is disclosed for the preparation of novel wholly aliphatic mono-and diisocyanates by thermal fracturing diurethanes that have been prepared by acid-catalyzed addition of alkyl carbamates to olefinic compounds, especially dienes. The compounds can be modified or used per se to produce curable compositions, e.g., with polyols.

EP 0 271 679 A2

Xerox Copy Centre

# ISOCYANATES DERIVED FROM DIENES AND PROCESS FOR THEIR PREPARATION

## FIELD OF THE INVENTION

This invention relates to the preparation of wholly aliphatic isocyanates by the thermal dissociation of corresponding urethanes that have been prepared from olefins especially diene compounds. More particularly, aliphatic mono-and diisocyanates are prepared by the uncatalyzed short resonance thermal fracturing of such corresponding urethanes.

The mono-and diisocyanates are useful as intermediates to prepare curing agents and as curing agents per se in resins and in other plastic applications.

## BACKGROUND OF THE INVENTION

Various methods have been employed to produce isocyanate compounds. It is known for example, that diamine or monamine derivatives can be reacted with phosgene at a temperature of about 100°C for about 28 hours. The by-product hydrogen chloride and excess phosgene are removed by blowing nitrogen through the liquid reaction mixture and recovering the desired mono-and diisocyanates. Drawbacks of processes such as this are the toxicity of the cyanic acid and phosgene, and corrosion problems associated with the byproduct hydrochloric acid.

The difficulties with direct phosgenations have led to the development of non-phosgenation routes, and these generally involve the pyrolytic thermolysis or cracking of carbamic acids, esters or urethanes.

Olefinic compounds have been investigated in several instances as a source or starting material for making carbamates.

In U.S. 3,290,350, Hoover discloses a process for making isocyanates by reacting isocyanic acid with vinylidene compounds, such as alpha-methyl styrene, isobutylene and the meta and para isomers of diisopropenyl benzene. This particular olefin route suffers the disadvantages that the yields are poor and that large amounts of olefin and isocyanic acid are lost through self-polymerization.

Stamm et al., U.S. 3,477,933, describe a process for preparing N-mono-substituted carbamic acid esters by the reaction of an N-monohalocarbamic acid ester with an unsaturated organic compound, such as cyclohexene, isobutylene, 4-vinyl-cyclohexene-1 and octadiene. In Col. 4, lines 21-24 of patent '933, it is reported that there can be obtained "excellent yields of diisocyanates by reacting N-monochlorocarbamic ester with dienes, e.g., the reaction of chlorourethane and octadiene. A major drawback of this teaching is the requirement for expensive reactants, i.e., halogenated N-carbamic acid esters.

More recently, Alexanian et al., U.S. 4,379,767, disclose a process for the production of tertiary benzyl isocyanates by reaction of the corresponding olefin, such as diisopropenyl benzene, or naphthalene and alpha-methyl styrene with a carbamoyl halide to form the benzyl halide followed by the reaction of the benzyl halide with excess isocyanic acid to form the isocyanate.

Singh et al., U.S. 4,439,616, describe a process for producing urethane intermediates by reacting corresponding olefins, such as diisopropenylbenzene (DIPEB), with carbamic acid esters. The tertiary aralkyl urethanes so formed are thermally cracked to isocyanates.

In U.S. 3,870,739, there are disclosed methods to prepare the mono-and diisocyanates of benzene, toluene, and xylene through non-catalytic short contact time thermal dissociation of aralkyl urethanes. However, these latter reactions produce isocyanates that are aromatic in nature, making the products less useful due to toxicity and poor light stability.

There has now been discovered a process for making novel wholly aliphatic mono-and diisocyanates by the thermal fracturing of diurethanes that have themselves been prepared by the addition of alkyl carbamates to olefinic compounds, preferably dienes, in the presence of acid catalyst.

## SUMMARY OF THE INVENTION

In accordance with the present invention is provided process for preparation of compounds selected from those of the formula

Y-R-X    (I)

or a cyclic derivative thereof wherein at least one of X and Y is -NCO and the remaining X or Y can be

$-NCO$, $-NHCO_2R^1$, aminocarboalkoxy or hydrogen wherein $R^1$ is alkyl of from about 1 to about 30, preferably 1 to 18, carbon atoms, and R is alkylene or alkenylene of from about 3 to about 5 carbon atoms, said process comprising thermally decomposing (fracturing) a urethane of the formula

G-R-Z (II)

wherein at least one of G or Z must be $-NHCO_2R^1$ wherein $R^1$ is as defined above, and the other can be -NCO or hydrogen, and R is as defined above, until formation of said compound (I) is substantially complete.

Further provided by the present invention are compounds selected from those of the formula

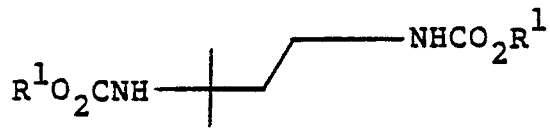

(III)

wherein $R^1$ is alkyl of from about 1 to about 30, preferably 1 to 18, carbon atoms.

Also contemplated are compounds selected from those of the formulae:

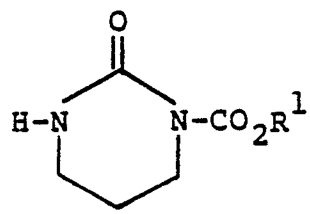

(IV)

or

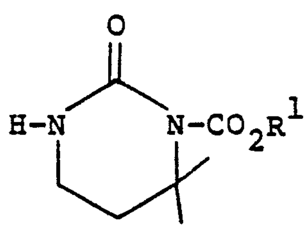

(V)

or mixtures of (IV) and (V) wherein $R^1$ is alkyl of from about 1 to about 30, preferably 1 to 18, carbon atoms.

Also provided in accordance with the invention herein are compounds selected from those of the formula

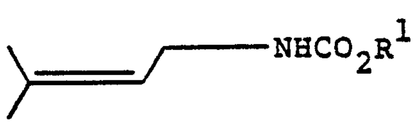

(VI)

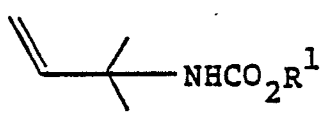

(VII)

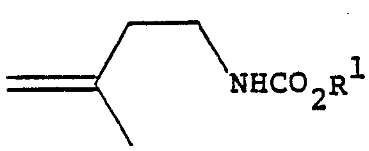

(VIII)

or a mixture of (VI), (VII) and (VIII) wherein R is alkyl of from about 1 to about 30, preferably 1 to 18, carbon atoms.

Also provided are compounds selected from those of the formulae:

Y-R-X

wherein at least one of X and Y is -NCO and the remaining X or Y is -NCO, $-NHCO_2R^1$ or hydrogen wherein $R^1$ is alkyl of from about 1 to about 30, preferably 1 to 18, carbon atoms, and R is alkylene or alkenylene of from about 3 to about 5 carbon atoms.

Also provided are compounds selected from those of the formulae:

OCN-R-NCO (IX)

$R^1-O_2CHN-R-NCO$ (X)

or

$OCN-R-NHCO_2R^1$ (XI)

or a mixture of (IX), (X) and (XI) wherein R is alkylene or alkenylene of from about 3 to 5 carbon atoms, and $R^1$ is alkyl of from about 1 to about 30 carbon atoms.

In addition there are provided compounds selected from those of the formulae:

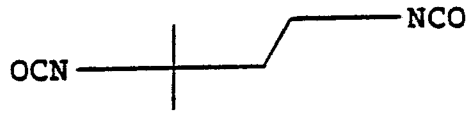 (XII)

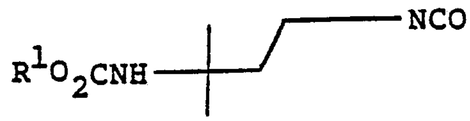 (XIII)

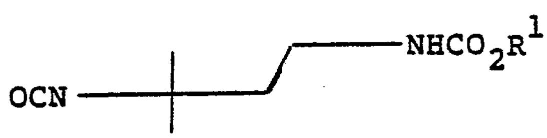 (XIV)

or a mixture of (XII), (XIII) and (XIV), wherein $R^1$ is alkyl of from about 1 to about 30 carbon atoms.

Still other compounds of the invention are selected from those of the formulae:

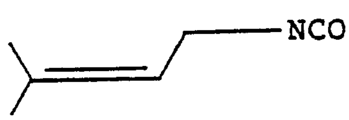 (XV)

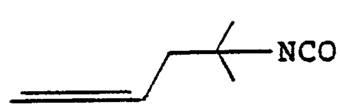 (XVI)

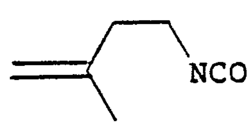 (XVII)

or a mixture of (XV), (XVI) and (XVII).

Among the features of this invention are curable compositions comprising:

(i) active hydrogen comounds, especially polyols, preferably hydroxy functional polyacrylates or polyesters; and

(ii) the novel isoprene diisocyanate above defined.

DETAILED DESCRIPTION OF THE INVENTION

The olefins useful as a starting material in accordance with this invention are alkylene or alkenylene compounds of from about 3 to about 5 carbon atoms including propene, 2-butene, 1-butene, isobutylene, 1-pentene, 2-pentene and the like. Special mention is made of diene compounds such as butadiene and isoprene.

The olefins and dienes described above may be reacted with alkyl carbamates of the formula:

$H_2N\text{-}CO_2R^1$

wherein $R^1$ is alky of from 1 to 30 carbon atoms, straight chain and branched, e.g., methyl, ethyl, propyl, 2-ethylhexyl, n-octadecyl, triacontyl, and the like. Especially preferred is the methyl ester of carbamic acid.

To carry out the condensation between the alkyl urethane and the olefin or diene, it is necessary to heat the components in the presence of an acidic catalyst. The preparation of the urethanes useful to make isocyanates in accordance with the present invention takes place at temperatures from 40°C up to 150°C in the presence of an acid such as sulfuric acid, boron trifluoride, toluene sulfonic acid, dodecyl benzene sulfonic acid, hydrocarbon sulfate esters, hydrochloric acid and other Lewis and Bronstead acids. A preferred catalyst is sulfuric acid. The reaction can take place in the absence of solvent or in the presence of solvents, such as methylene dichloride, toluene, xylene, chlorobenzene, and the like.

The proportion of alkyl carbamate to the olefin or diene compound is typically in excess of stoichiometric, ranging from 100 mole % to 500 mole %.

The amount of catalyst required to promote the addition of alkyl carbamate to olefin or diene is not critical and can be varied widely. The amount is typically from 0.5 to 50 mole %, and preferably about 1 to 5 mole %.

Preferably the catalyst is dissolved or suspended in the warm reaction mixture and the olefin or diene is slowly added. When the reaction is complete, the time generally being from about 2 hours to about 26 hours, the mixture is treated to remove or neutralize the catalyst by adjusting the pH to 9, e.g., by the addition of 2N sodium hydroxide solution. Then the unreacted materials and solvent, if used are removed, e.g., by distillation, leaving the condensation product as a residue. Purification can be effected, e.g., by column separation using, e.g., methylene chloride, as an eluent.

The reaction can take place in the absence or in the presence of solvents, such as methanol, ethanol, acetic acid, methylene dichloride, ethylene dichloride, chlorobenzene, and so forth.

The aliphatic urethanes derived from olefins or dienes form the corresponding isocyanates by thermal cracking while splitting off the corresponding alkanol. With 1,3-butadiene used as the starting material, the ultimate compounds are of the formulae:

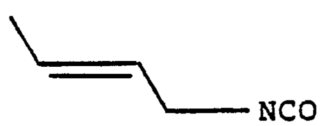

and

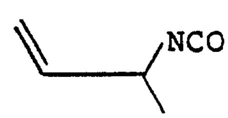

With isoprene used as the starting material, the ultimate compounds are of the formulae:

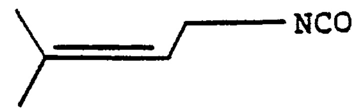

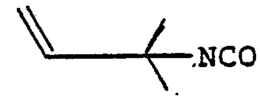

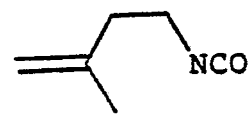

and

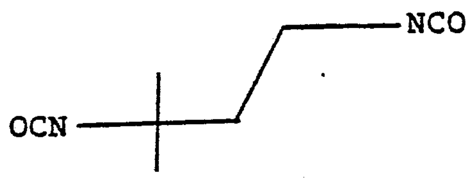

In many cases, the alcohol, preferably methanol, can usefully be recycled with urea or isocyanic acid to form methyl carbamate, which can be reacted with further olefin or diene.

In cracking the urethane esters to form the corresponding isocyanates, the acidic catalyst must be removed or neutralized, for example with calcium oxide, sodium carbonate, sodium hydroxide and the like, which is followed by solvent-free or in high boiling solvents cracking of the urethane ester such as hexadecane, diphenyl ether, diisopropyl naphthalene and the like. Cracking takes place at temperatures on the order of 300°C to about 650°C, preferably 350 to 600°C, splitting off the alcohol to yield the corresponding isocyanates.

Thermolysis or cracking of the urethane esters may be conducted by adding a solid melt or solution of the urethane ester in a solvent through a tube which may comprise a variety of materials including glass and metal. The tube can contain a variety of materials as well including glass beads or chips, metal pieces, e.g., stainless steel or copper.

Typically, the tube is heated to temperatures of around 400-550°C, preferably 480-520°C. Pressures for the cracking system can vary widely, however, best selectivities are obtained with pressures between 50 and 70 mm Hg. Time for cracking can also vary widely but it is generally preferred to keep the resonance time or contact time to a minimum so as to reduce the occurrence of undesirable side reactions.

The isocyanates can be separated from the alcohol, e.g., methanol by conventional, e.g., distillation, as will be exemplified in the next section, methods. The isocyanate products are typically obtained as liquids, ranging from colorless to slightly yellow in color at room temperatures.

Because of their unique highly branched structure, the mono-and diisocyantes of this invention should have better physical properties when used as reactants with polyester polyols, polyether polyols and the like, to make polyurethanes useful in coating applications. In particular, the isoprene diisocyantes whose preparation will be hereinafter exemplified in the next section, are useful in preparing surface coatings with acrylics and polyesters. For example, if, in accordance with known techniques, coatings compositions are made by mixing polymers with effective amounts, e.g., 0.5-5 -NCO/-OH of the diisocyanates optionally in a hydrocarbon solvent, preferably with a catalyst, e.g., 1% of a tin compound, and curing, e.g., at 60-150°C. for 1 minute to 1 hour, hard, solvent-resistant coatings will be obtained.

DESCRIPTION OF THE PREFERRED EMBODIMENTS

The following examples illustrate the novel processes and novel compounds of the present invention. They are not to be construed to limit the claims in any manner whatsoever.

(a) Preparation of 1,3-diaminocarbomethoxy-3-methylbutane. 400 g (6.15 mol) of isoprene, and 2,768 g (37 mol) of methyl carbamate were reacted in a flask in the presence of 121 g (1.23 mol) of sulfuric catalyst. The product, 1,3-diaminocarbomethoxy-3-methylbutane, was obtained in 38-45 % yield as a yellow liquid, b.p. 155°-160°C at 0.5 mm Hg.

(b) The product in (a), 55 parts, was melted in a pressure equalizer addition funnel. The liquid was passed through a pyrex tube containing 316-stainless steel sheathings which was maintained at 500°C at a pressure of 35 mm Hg. The rate of addition varied between 10 and 17 g of liquid per hour. A crude liquid was obtained which was fractionally distilled to provide 29 parts of 1,3-diisocyanato-3-methyl butane, and 4 parts of a mixture of 1-amino-carbomethoxy-3-isocyanato-3-methylene and 3-aminocarbomethoxy-1-isocyanato-3-methylbutane of the formulae:

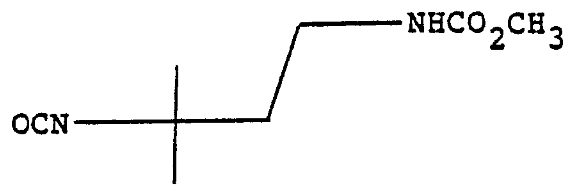

and

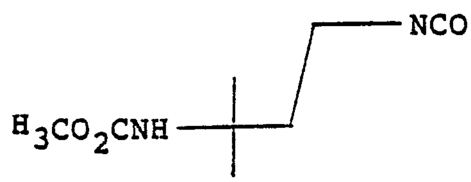

EXAMPLE 2

The procedure of Example 1 was followed except that in (b), the pyrex tube contained glass chips. Analysis indicated that a mixture of products were obtained.

EXAMPLE 3

The procedure of Example 1 was followed except that in (b), the pyrex tube contained copper metal sheet. Analysis indicated that a mixture of products were obtained.

EXAMPLE 4

The procedure of Example 1 was repeated except that in (b), 1,3-diaminocarbomethoxy-3-methylbutane was dissolved in 100 parts of methylene chloride solvent.

EXAMPLE 5

(a) Preparation of Urethane Allophantes. The urethane allophantes of the formula

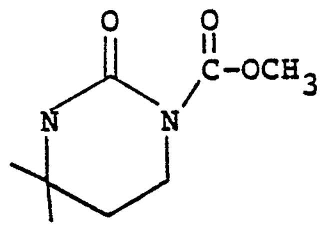

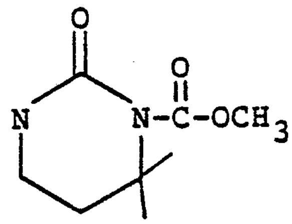

were prepared by heating 100 g of III in the presence of (1.5 mol%) of p-toluenesulfonic acid catalyst to 180°C at 120 mm Hg.

EXAMPLE 6

The procedure of Example 5(a) and (b) was followed except that in (b), the urethane allophantes were dissolved in toluene solvent.

EXAMPLE 7

(a) Preparation of Isoprene Monourethanes . One mole of sulfuric acid was added slowly to a solution containing 15 moles of methyl urethane and 600 mls of 1,2-dichloroethane. The solution was then heated to 60°C and 5 moles of isoprene were added at a rate such that it refluxed slowly in the reaction mixture. After 20 hours at 60°C the reaction was cooled to room temperature and diluted with 1 of methylene chloride. The solution was then neutralized with 2N NaOH until the pH was greater than 8. The organic layer was separated, washed with $H_2O$ and dried over magnesium sulfate. The resulting mixture was then fractionally distilled at reduced pressure. Typical yields of product were 45% based on isoprene charged.

(b) Preparation of Isoprenemonoisocyanates. The procedure of Example 1(b) was repeated except a mixture of isoprene monourethanes of the formulae

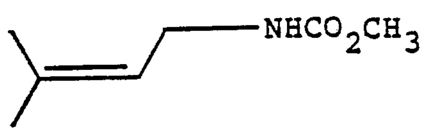

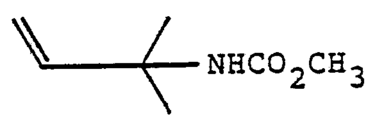

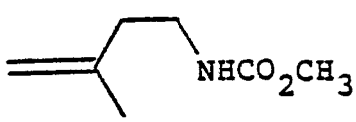

were used. The products were purified by fractional distillation.

EXAMPLE 8

(a) The procedure of Example 7 was repeated except that a temperature of 70°C was used instead of 60°C. After distillation, the isoprene diurethane was purified by crystallization from methylene chloride and hexane. Analysis indicated that a yield of product of approximately 38%.

(b) Preparation of Isoprene diisocyantes. If the procedure of Example 1(b) is followed except that a mixture of isoprene diisocyanates is cracked at temperatures above 200°C instead of 500°C, and the pressure used is 60 mm Hg and not 35 mm Hg, the isocyanate products will be obtained after re-distillation.

EXAMPLE 9

The procedure of Example 8 is followed except that an acid catalyst, p-toluenesulfonic acid, is added in step (b) to the reaction mixture.

EXAMPLE 10

The procedure of Example 8 was repeated except that a basic catalyst, DABCO®, was added in step (b) to the reaction mixture.

EXAMPLE 11

Methyl carbamate (1 mole) and sulfuric acid (0.1-0.5 mole) were placed in a glass lined autoclave. The auto clave was sealed and 0.5 to 1.0 mole of butadiene was added. The autoclave was heated to 100° for 4-12 hours (maximum pressure = 150-400 psig). The autoclave was cooled and vented, and the organic product was isolated to give a crude product which was analyzed by gas chromatography.

Two isomeric carbamates were produced in approximately equal amounts:

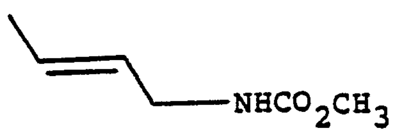

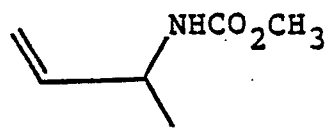

The total yield was 30 to 40%.

EXAMPLE 12

The procedure of Example 11 was followed substituting, however, a sulfonic acid ion exchange resin Amberlyst® 15 for sulfuric acid. Substantially the same results were obtained.

EXAMPLE 13

A curable composition is made comprising a hydroxy functional acrylic acid and the isoprene diisocyanate (IDI) of Example 1, step (b). If a copolymer of hydroxyethyl acrylate with other acrylics (G-CURE® 867) and IDI at 50% non-volatiles in a hydrocarbon solvent, the -NCO/-OH ratio being 1.1/1.0 is treated with 1.0% (TRS) of a tin catalyst, UL-28, and spread on a 1200 S aluminum substrate and cured for 20 minutes at 60°, 80° and 100°C, cured coatings will be obtained in accordance with this invention.

EXAMPLE 14

A curable composition is made comprising a hydroxy functional acrylic acid and the isoprene diisocyanate (IDI) of Example 1, step (b). If a hydroxy functional polyester (MULTRON® 221-75) and IDI at 60% non-volatiles in a hydrocarbon solvent, the -NCO/-OH ratio being 1.1/1.0 is treated with 1.0% (TRS) of a tin catalyst, UL-28, and spread on a 1200 S aluminum substrate and cured for 20 minutes at 60°, 80° and 100°C, a cured coating in accordance with this invention will be obtained.

Many variations will suggest themselves to those skilled in the art, in light of the above detailed description. For example, instead of sulfuric acid, or p-toluenesulfonic acid as catalyst, zinc chloride and aluminum chloride could be used.

All such obvious variations are within the full intended scope of the appended claims.

## Claims

1. A process for preparation of compounds selected from those of the formula

Y-R-X (I)

or a cyclization product thereof wherein at least one of X and Y is -NCO and the remaining X or Y can be -NCO, $-NHCO_2R^1$, aminocarboalkoxy or hydrogen wherein $R^1$ is alkyl of from about 1 to about 30 carbon atoms, and R is alkylene or alkenylene of from about 3 to about 5 carbon atoms, said process comprising thermally decomposing (fracturing) a urethane of the formula

G-R-Z (II)

wherein at 'east one of G or Z must be $-NHCO_2R^1$ wherein $R^1$ is as defined above, and the other can be -NCO or hydrogen, and R is as defined above, until formation of said compound (I) is substantially complete.

2. A process as defined in Claim 1 wherein the urethane is thermally decomposed at a temperature of from about 300°C to about 650°C.

3. A process as defined in Claim 1 wherein said urethane is dissolved in an organic solvent before thermally decomposing.

4. A process as defined in Claim 3 wherein said solvent is selected from methylene chloride, toluene, benzene, chlorobenzene or a mixture of any of the foregoing.

5. A process as defined in Claim 4 wherein said solvent is methylene chloride or toluene.

6. A process as defined in Claim 1 wherein said urethane is

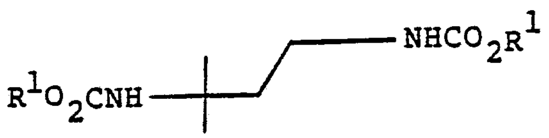

(III)

7. A process as defined in Claim 1 wherein said urethane is 1,3-diaminocarbomethoxy-3-methylbutane.

8. A process as defined in Claim 1 wherein said urethane is a cyclic product of the formulae:

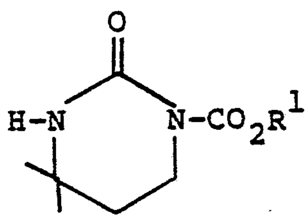

(IV)

or

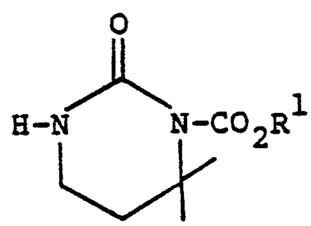

(V)

or a mixture of (III) and (IV).

9. A process as defined in Claim 8 wherein said urethane is

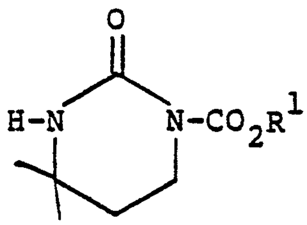

(IV)

10. A process as defined in Claim 8 wherein said urethane is

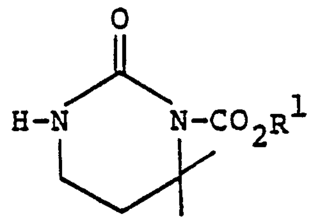

(V)

11. A process as defined in Claim 1 wherein said urethane is selected from those of the formuale:

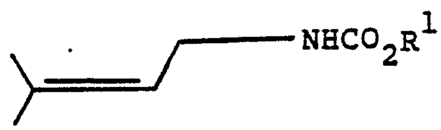

(VI)

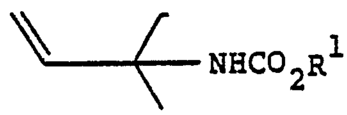

(VII)

or

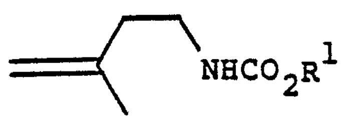

(VIII)

or a mixture of (VI), (VII) and (VIII).

12. A compound selected from those of the formula

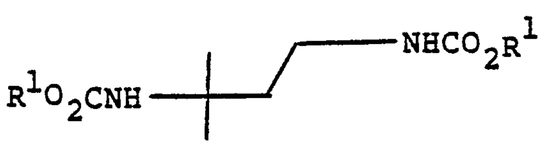

(III)

wherein $R^1$ is alkyl of from about 1 to about 18 carbon atoms.

13. A compound as defined in Claim 12 wherein $R^1$ is methyl.

14. A compound selected from those of the formulae:

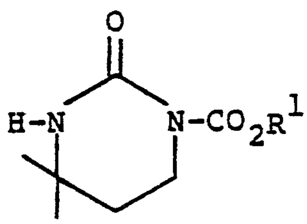

(IV)

or

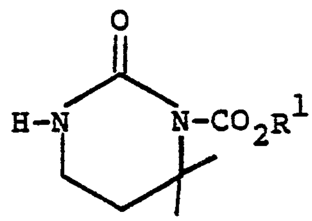

(V)

or mixtures of (IV) and (V) wherein $R^1$ is alkyl of from about 1 to about 30 carbon atoms.

15. A compound as defined in Claim 14 wherein $R^1$ is methyl.

16. A compound selected from those of the formulae:

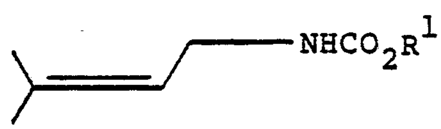

(VI)

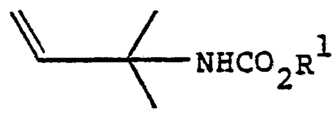

(VII)

or

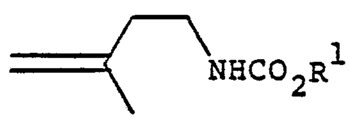

(VIII)

or a mixture of (VI), (VII) and (VIII) wherein R is alkyl of from about 1 to about 30 carbon atoms.

17. A compound as defined in Claim 16 wherein $R^1$ is methyl.

18. A compound selected from those of the formula:

Y-R-X (I)

or a cyclic derivative thereof wherein at least one of X and Y is -NCO and the remaining X or Y is -NCO, $-NHCO_2R^1$ or hydrogen wherein $R^1$ is alkyl of from about 1 to about 30 carbon atoms, and R is alkylene or alkenylene of from about 3 to about 5 carbon atoms.

19. A compound selected from those of the formulae:

OCN-R-NCO (IX)

$R^1$-$O_2$CHN-R-NCO (X)

or

OCN-R-$NHCO_2R^1$ (XI)

or a mixture of (X), (XI) and (XII) wherein R is alkylene or alkylene of from about 3 to about 5 carbon atoms, and $R^1$ is alkyl of from about 1 to about 18 carbon atoms.

20. A compound selected from those of the formulae:

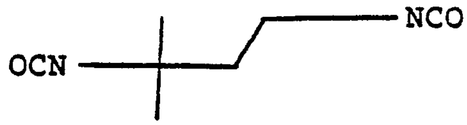

(XII)

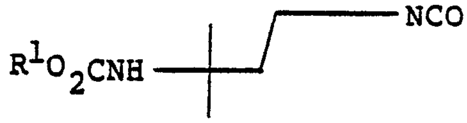

(XIII)

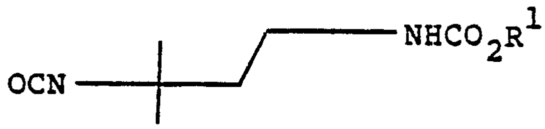

(XIV)

or a mixture of (XII), (XIII) and (XIV), wherein $R^1$ is alkyl of from about 1 to about 30 carbon atoms.

21. A compound selected from those of the formulae:

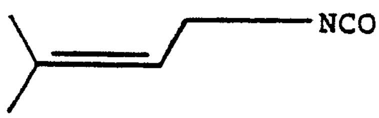

(XV)

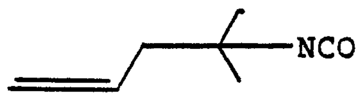

(XVI)

or

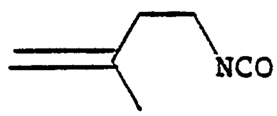

(XVII)

or a mixture of (XV), (XVI) and (XVII).

22. A curable composition comprising:

(i) a polyol; and

(ii) a diisocyanate of the formula:

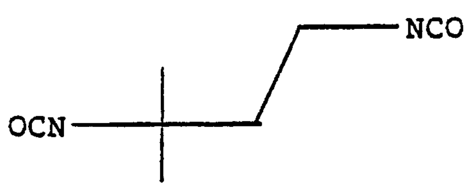

.

23. A curable composition as defined in Claim 22 wherein polyol (i) is a hydroxy functional polyacrylate.

24. A curable composition as defined in Claim 22 wherein polyol (i) is a hydroxy functional polyester.